(19) Europäisches Patentamt

European Patent Office

Office européen des brevets

(11) Veröffentlichungsnummer: **0 286 869 B1**

(12) **EUROPÄISCHE PATENTSCHRIFT**

(45) Veröffentlichungstag der Patentschrift: **11.11.92**

(21) Anmeldenummer: **88104450.7**

(22) Anmeldetag: **21.03.88**

(51) Int. Cl.⁵: **A61K 35/74**

(54) **Verfahren zur Herstellung von Zellwandkomponenten aus Halobakterien und deren Verwendung als Arzneimittel.**

(30) Priorität: **31.03.87 DE 3710606**
**19.05.87 DE 3716669**

(43) Veröffentlichungstag der Anmeldung:
**19.10.88 Patentblatt 88/42**

(45) Bekanntmachung des Hinweises auf die
Patenterteilung:
**11.11.92 Patentblatt 92/46**

(84) Benannte Vertragsstaaten:
**AT BE CH DE ES FR GB IT LI NL SE**

(56) Entgegenhaltungen:
**EP-A- 0 206 942**
**EP-A- 0 237 401**

**CHEMICAL ABSTRACTS, Band 84, Nr. 21, 24.
Mai 1976, Seite 197, Zusammenfassung Nr.
146473n, Columbus, Ohio, US; M.F. MESCHER
et al.: "Purification and characterization of a
prokaryotic glycoprotein from the cell envelope of Halobacterium salinarium", & J.
BIOL. CHEM. 1976, 251(7), 2005-14**

(73) Patentinhaber: **BAYER AG**

**W-5090 Leverkusen 1 Bayerwerk(DE)**

(72) Erfinder: **Baumgarten, Jörg, Dr.**
**Henselweg 13**
**W-5600 Wuppertal 1(DE)**
Erfinder: **Brunner, Helmut, Prof. Dr.**
**Am Rohm 111**
**W-5600 Wuppertal 1(DE)**
Erfinder: **Flesch, Inge, Dr.**
**Im Starkteld 16/2**
**W-7900 Neu-Ulm(DE)**
Erfinder: **Hildebrand, Heinz, Dr.**
**Molecular Diagnostic INC. 400 Morgan Lane**
**West Haven CT 06516(US)**
Erfinder: **Piel, Norbert, Dr.**
**Millrather Weg 74**
**W-4006 Erkrath(DE)**
Erfinder: **Sperzel, Michael, Dr.**
**Siegfriedstrasse 39**
**W-5600 Wuppertal 1(DE)**

CHEMICAL ABSTRACTS, Band 102, Nr. 13, 1. April 1985, Seite 374, Zusammenfassung Nr. 109535d, Columbus, Ohio, US; E.N. SPUDICH et al.: "Biochemical characterization of halorhodopsin in native membranes", & J. BIOL. CHEM. 1985, 260(2), 1208-12

BIOLOGICAL ABSTRACTS, Band 80, 1985, Zusammenfassung Nr. 53059, Philadelphia, US; J. BARIBEAU et al.: "Isolation, purification and partial characterization of stable forms of monomeric bacteriorhodopsin in lauryl sucrose", & CAN. J. BIOCHEM. CELL. BIOL., 63(4), 305-312

CHEMICAL ABSTRACTS, Band 91, Nr. 19, 5. November 1979, Seite 226, Zusammenfassung Nr. 153117x, Columbus, Ohio, US; L.L. YANG et al.: "Purification and partial characterization of a procaryotic glycoprotein from the plasma membrane of Thermoplasma acidophilum", & BIOCHIM. BIOPHYS. ACTA 1979, 556(2), 265-77

## Beschreibung

Die Erfindung betrifft ein Verfahren zur Herstellung von Zellwandkomponenten aus Halobakterien, Arzneimittel enthaltend solche Zellwandkomponenten und deren Verwendung zur Infektabwehr.

Es wurde nun gefunden, daß Proteine, Glycoproteine und Glycopeptide, die aus Halobakterien gewonnen werden können, zur Infekttionsabwehr, insbesondere als immunologisch aktive Präparate verwendet werden können.

Halobakterien, die zu den Archaebakterien gehören, werden ausführlich in The Bacteria, A Treatise On Structure And Function, Vol. VIII, Archaebacteria, Academic Press Inc., 1985; Archaebacteria, edited by Otto Kandler, Gustav Fischer Verlag, Stuttgart, New York 1982 und Archaebacteria, J. Mol . Evol. 11, 245-252 (1978), C.R. Woese et al. beschrieben.

Die Zellhüllenchemie der Archaebakterien war eines der ersten phenotypischen Charakteristika zur Abgrenzung der Archaebakterien von den normalen Eubakterien. Die normalen Zellhüllenkomponenten des Mureins der Eubakterien werden bei Archaebakterien nicht gefunden. Dafür kommen Zellwandpolymere wie Pseudomurein, Heteropolysaccharide oder Glycoproteine vor. Die meisten Gram-negativen Archaebakterien bilden als Zellhülle nur eine Oberflächenschicht (S-Layer) aus regelmäßig angeordneten Glycoproteinen aus.

Diese S-Layer kommen bei Thermoacidophilen, bei Halobakterien und bei Gram-negativen Methanogenen vor. Außerdem bestehen Scheiden, in die bestimmte "Methanogene Archaebakterien" eingeschlossen sind, aus Glycoproteinen (The Bacteria,Vol. 8, Chapter 9: "The Envelopes of Archaebakteria", Otto Kandler, Helmut König.)

Gegenstand der Erfindung ist ein Verfahren zur Herstellung von Arzneimitteln enthaltend Zellwandkomponenten aus Zellkulturen von Halobakterien, das dadurch gekennzeichnet ist, daß man Halobakterien anzüchtet, die Zellwandkomponenten der Halobakterien isoliert, die Zellwandkomponenten enzymatisch abbaut und die Abbauprodukte reinigt und isoliert, wobei die Auswahl der Fraktionen bei den einzelnen Reinigungsschritten ausschließlich aufgrund der immunstimulierenden Aktivität der Fraktionen erfolgt.

Die verfahrensgemäß hergestellten Produkte sind ebenfalls Gegenstand der Erfindung.

Verwendet werden für das erfindungsgemäße Verfahren als Ausgangsbakterium für die Zellkulturen Halobacterium salinarium (DSM 668) oder Halobacterium halobium (DSM 671) verwendet.

Weiterer Gegenstand der Erfindung sind Arzneimittel enthaltende Zellwandkomponenten aus Kulturen bzw. Zellwänden von Archaebakterien, insbesondere Arzneimittel zur Infektionsabwehr, die solche Zellwandkomponenten aus Kulturen bzw. Zellwänden von Archaebakterien enthalten.

Die Verwendung von Zellwandkomponenten aus Kulturen bzw. aus Zellwänden von Archaebakterien zur Herstellung von Arzneimitteln ist ebenfalls Gegenstand der Erfindung.

Bevorzugt ist die Verwendung von Zellwandkomponenten aus Kulturen bzw. aus Zellwänden von Archaebakterien zur Herstellung von zur stimulierenden Infektionsabwehr geeigneten Arzneimitteln.

Die Zellwandkomponenten sind Proteine, Glycoproteine und/oder Glycopeptide.

Das erfindungsgemäße Verfahren wird beispielhaft wie folgt ausgeführt:

Zur Isolierung der Zellwandkomponenten mit immunstimulierender Wirkung wurden die Halobakterien in üblicher Nährlösung wie im DSM Catalogue of Strains beschrieben angezogen.

Die Isolierung der Glycoproteine aus den Zellhüllen erfolgt nach Delipidierung der Zellen mit einer Mischung aus Chloroform-Methanol durch Phenolextraktion.

Alternativ können die Zellhüllen durch ionische und nichtionische Detergention z.B. SDS solubilisiert werden. Darüber hinaus können die Zellwandproteine direkt ohne vorherige Solubilisierung enzymatisch degradiert werden. Die solubilisierten Zellhüllen werden anschließend einer enzymatischen Spaltung unterworfen. Der enzymatische Abbau von Glycoproteinen aus Zellwänden von Archaebakterien kann durch Trypsin, Subtilisin, Pronase, Chymotrypsin, Pepsin, Proteinase K, Papain, Carboxypeptidase A, -B, -P, -Y, Elastase, Pepsin und Thermolysin sowie durch Kombination der verschiedenen Enzyme erfolgen.

Die Aufarbeitung der enzymatischen Spaltprodukte und/oder nativen Glycoproteine erfolgt durch Trennung nach dem Molekulargewicht, durch Ionenaustauscherchromatographie und/oder hydrophober Chromatographie.

Sowohl die Glycoproteine aus den Zellhüllen der Halobakterien als auch Fraktionen (Glycopeptide) die sich aus der Aufreinigung nach den enzymatischen Spaltungen ergaben, zeigten immunstimulierende Eigenschaften.

Beispiele für die Gewinnung immunstimulierender Substanzen

Beispiel 1

Anzucht von Halobacterium salinarium (DSM 668) und Halobacterium halobium (DSM 671)

Die Nährlösung zur Anzucht von Halobacterium hatte folgende Zusammensetzung:
7,5 g Casaminoacids; 10 g Hefeextrakt; 3,0 g Na-Citrat; 2 g KCl; 20 g $MgSO_4 \cdot 7 H_2O$; 0,05 g $FeSO_4 \cdot 7 H_2O$; 0,2 mg $MnSO_4 \cdot H_2O$; 220 g Meersalz; 1 l $H_2O$.

Eine Blasensäule mit 90 Liter Nährlösung wurde mit 10 Liter eine 4 Tage alten Vorkultur von Halobacterium beimpft und unter Belüftung mit 2,5 $m^3$/h Luft für 8 Tage bei 37°C inkubiert. Die angezogenen Zellen wurden in der Cepa-Stabzentrifuge (Dauer der Zentrifugation: 2 h) geerntet. Eine Anzucht mit Halobacterium salinarium brachte 1.470 g Zellfeuchtgewicht. Eine Anzucht mit Halobacterium halobium brachte 2.215 g Zellfeuchtmasse.

Beispiel 2

Isolierung des Zellwandglycoproteins aus Halobacterium salinarium

90 l Kulturbrühe wurden in einer Durchlaufzentrifuge geklärt (Typ Cepa-Stabzentrifuge); der klare Durchlauf wurde verworfen. Die Zellen (1.470 g Feuchtmasse) wurden in 3 l Wasser suspendiert und mit einer Passage durch die Dyno-Mill aufgeschlossen (Flußrate 0,8 l/h; Korngröße der Glasperlen ca. 0,1 mm). Zur Delipidierung rührte man dies in 60 l einer Mischung von Dichlormethan und Methanol (2:1), rührte 3 h und ließ über Nacht absitzen. Das noch leicht rötliche Sediment wurde mit 5 bis 10 l der gleichen Mischung gewaschen (bis es eine helle weiße Farbe hatte) und im Vakuumtrockenschrank bei Raumtemperatur getrocknet.
Ausbeute 743,6 g.

Zur weiteren Anreicherung der Zellwandkomponenten wurde diese Fraktion einer Phenolextraktion unterzogen, indem sie in 5,0 l 0,05 M Tris/HCl-Puffer,pH 7,2, suspendiert wurde unter Zusatz von 3.960 g Phenol und 45 g KCl.

Nach 2,5 h Mischen im Kühlraum wurde zur Klärung zentrifugiert (50 min., 2.000 rpm) und die Oberphase gegen destilliertes Wasser dialysiert (ca. 3 Tage, bis kein Phenolgeruch mehr wahrnehmbar). Unlösliche Bestandteile wurden abzentrifugiert und der klare Überstand lyophilisiert.
Ausbeute: 2,21 g.

Beispiel 3

Isolierung von Glycopeptiden aus Glycoprotein

A. Tryptischer Abbau der Glycoproteinfraktion

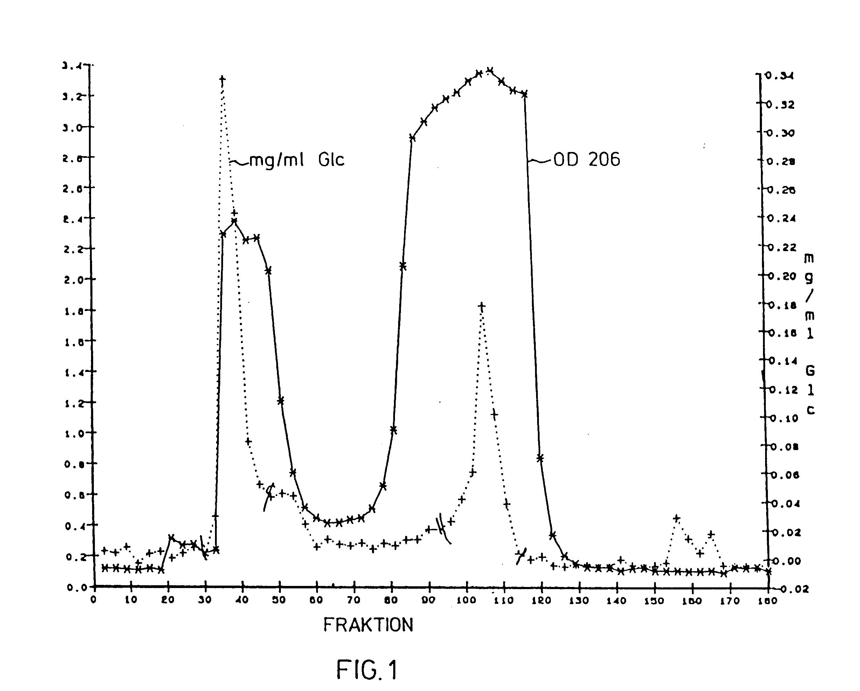

2,21 g der Zellwandkomponentenfraktion wurden in 140 ml einer sterilfiltrierten Lösung von 210 mg Trypsin, 240 mg Natriumazid in 0,05 M Tris-HCl-Puffer, PH 7,2, gelöst und 24 h bei 37°C inkubiert. Der Ansatz wurde dann portionsweise (8 Portionen) über Ultrogel AcA 44 chromatographiert (Flow 40 ml/h; Eluat, Wasser), wobei das Eluat mittels Anthrontest auf kohlenhydrathaltige Fraktionen untersucht wurde. Diese wurden vereinigt und lyophilisiert (s. Abbildung 1; Elutionsdiagramm einer Ultrogel-Säulenchromatographie).
Ausbeute: Fraktion I 228,6 mg; Fraktion II 1.526,7 mg.
Fraktion II hatte immunstimulierende Aktivität.

B. Subtilisinabbau der kohlenhydrathaltigen Fraktion

1.526,7 mg Fraktion II aus der Ultrogeltrennung wurden in einer steril filtrierten Lösung von 27 mg Natriumazid, 153 mg Subtilisin in 134 ml Tris-HCl-Puffer (0,05 M, pH 8,0) gelöst und 24 h bei 37°C inkubiert. Daraufhin wurde die Lösung entweder sofort weiter aufgearbeitet (s. Abschnitt C) oder bei -20°C aufbewahrt.

C. Reinigung über CM-Sephadex und Biogel P-2

CM-Sephadex wurde mit NaCl (2 M Lösung) in die $Na^+$-Form überführt und mit Na-Phosphat auf pH

EP 0 286 869 B1

7,0 eingestellt (5 mMol/l). Die klarfiltrierte Lösung aus B. (s. oben) wurde mit 16,7 ml/min über die Säule gepumpt, danach spülte man die Säule noch mit 2 Bettvolumina Puffer (1,2 l). Eluat und Waschpuffer wurden vereinigt und lyophilisiert.

Das Lyophilisat wurde ausgewogen und portionsweise in sterilfiltriertem Wasser gelöst, um über eine Säule mit Biogel-P 2 chromographiert zu werden.

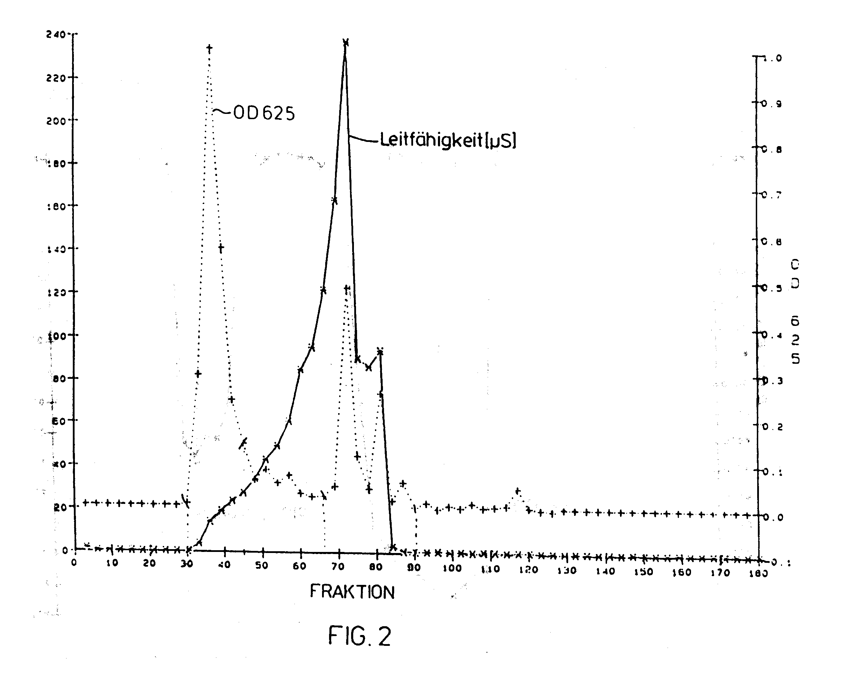

Säule 2,5 x 100 cm; Bettvolumen 450 ml; Flow 40 ml/h; Fraktion 4 ml ; Eluent: Wasser; Detektion Leitfähigkeit und Anthrontest (Elutionsprofil s. Abb. 2).

Entsprechend dem Elutionsprofil des Anthrontestes wurden die kohlenhydrathaltigen Fraktionen vereinigt und lyophilisiert.

| Fraktion I | 179,4 mg |
| Fraktion II | 1.223 mg |

Fraktion I ist die Zellwandkomponentenfraktion mit der höchsten immunstimulierenden Wirkung.

D. Reinigung über QUAE-Sephadex A-25

Fraktion I der P-2 Säule wird in 4,5 ml destilliertem $H_2O$ aufgenommen und auf eine mit 0,1 M Tris-HCl-Puffer, pH 6,8, äquilibrierte QUAE-Sephadex A-25-Säule (100 ml Säulenvolumen) aufgetragen und nach Waschen mit 100 ml Aufgabepuffer mit einem linearen Salzgradienten von 0 → 1 M NaCl in 0,1 M Tris-HCl-Puffer, pH 6,8, eluiert. Hexosen wurden mit dem Anthrontest und Uronsäuren mit Carbazol in den einzelnen Fraktionen detektiert. Die vereinigten aktiven Fraktionen wurden lyophilisiert und durch Gelchromatographie an einer Biogel P-2-Säule (2,5 x 100 cm) entsalzt. Ausbeute: 92,1 mg.
Diese Substanz zeigte immunstimulierende Wirkung.

Im Anschluß an Beispiel 2 lassen sich auch alternativ die folgenden Schritte zur Gewinnung immunstimulierender Zellwandkomponenten durchführen.

A'. Pronaseabbau der Glykoproteinfraktion

3,0 g der Glykoproteinfraktion aus Halobacterium salinarium wurden in 150 ml einer Lösung aus 0,1 M Tris-HCl-Puffer, pH 7,5, 10 mM $CaCl_2$ und 0,01 Gew.-% Natriumazid gelöst, zentrifugiert und mit 0.75 g Pronase E 15 h bei 39°C inkubiert. Anschließend wurden weitere 0,75 g Pronase E zugegeben und 9 h bei 56°C inkubiert. Der Ansatz wurde dann mit 220 ml Dowex W 50 - X 8 ($H^+$-Form) im Batchverfahren 30 Min. versetzt, abgesaugt und mit 600 ml und 800 ml $H_2O$ gewaschen. Diese drei Fraktionen wurden vereinigt, durch Zugabe von festem $NaHCO_3$ neutralisiert und lyophilisiert.

Der Durchlauf sowie die beiden Waschphasen wurden mittels Anthrontest auf kohlenhydrathaltige Substanzen untersucht.

| Ausbeute: | |
| --- | --- |
| Durchlauf: | 2,67 g, |
| Waschphase | I: 0,13 g, |
| Waschphase | II: 0,02 g. |

B'. Reinigung über Biogel P-10

Das Lyophilisat aus A'. wurde in $H_2O$ aufgenommen und portionsweise (2 Portionen) über eine Biogel P-10-Säule (2,5 x 100 cm; Flow: 40 ml/h) mit Wasser als Eluationsmittel chromatographiert. Das Eluat wurde mittels Anthrontest auf Hexosen- und Carbazoltest auf Uronsäurenhaltige Fraktionen (Fraktionsvolumen je 4 ml) getestet.
Fraktionen 25 - 55 und Fraktionen 70 - 130 wurden vereinigt und lyophilisiert.

5

| Ausbeute: | |
|---|---|
| Fraktion I | 0,4 g |
| Fraktion II | 2,4 g |

Fraktion II enthielt die Substanz mit der höchsten immunstimulierenden Wirkung.

C'. Reinigung über Biogel P-2

Die lyophilisierte Fraktion II aus B'. wurde in destilliertem $H_2O$ aufgenommen und portionsweise über eine Biogel P-2-Säule (2,5 x 100 cm) mit Wasser als Eluationsmittel chromatographiert. Es wurden Fraktionen von je 4 ml genommen und anhand der Farbteste mit Anthron auf Hexosen und Carbazol auf Uronsäuren sowie die Leitfähigkeit detektiert.
Fraktionen 30 - 50 und 51 - 65 sowie 66 - 97 wurden vereinigt und lyophilisiert.

| Ausbeute: | |
|---|---|
| Fraktion I | 320,8 mg |
| Fraktion II | 74,0 mg |
| Fraktion III | 826,7 mg |

Fraktion I ist die Glykopeptid-Fraktion mit der höchsten immunstimulierenden Wirkung.
Nach einem weiteren Beispiel wird das erfindungsgemäße Verfahren wie folgt ausgeführt:

Beispiel 4

A. Isolierung von Glycoprotein aus Halobacterium halobium

2.215 g Feuchtmasse von Halobacterium halobium wurde in 3 l Wasser suspendiert und mit einer Passage durch die Dyno-Mill aufgeschlossen (Flußrate 0,8 l/h; Korngröße der Glasperlen ca. 0,1 m). Zur Entfettung wurden die Zellen 2 x in 33 l Methanol-Chloroform-Mischung 2 : 1 drei Stunden gerührt. Nach dem Absetzen der Zellen über Nacht wurde das Methanol-Chloroform-Gemisch abdekantiert. Die entfetteten Zellen wurden 48 h bei 30 ° C unter Vakuum getrocknet.
Ausbeute: 630 g
Anschließend wurde mit 5,0 l 0,05 M Trispufferlösung + 0,5 % KCl, pH 7,2, die 44 % Phenol enthielt, 3 h extrahiert. Das Gemisch wurde in der Hettich Zentrifuge eine Stunde bei 2.000 U/min zentrifugiert. Die obere wässrige Phase wurde abgenommen und 72 h gegen Wasser dialysiert. Unlösliche Bestandteile wurden abzentrifugiert und der klare Überstand lyophilisiert.
Ausbeute: 4,68 g.

B. Aufarbeitung der Glykoproteine aus Halobacterium halobium

4,6 g des Phenolextraktes wurden in 314 ml 0,1 M Tris-HCl-Puffer, pH 7,5, und 10 mM $CaCl_2$ gelöst, 1,575 g Pronase E. zugefügt und 12 h bei 39 ° C inkubiert. Anschließend wurden erneut 1,575 g Pronase E. zugegeben und nochmals 12 h bei 56 ° C geschüttelt.
Die so behandelte Lösung wird sterilfiltriert und über eine Dowex W50-X8-Säule (370 ml Säulenvolumen) mit einem Flow von 50 ml/h gepumpt.
Die Säule wurde zweimal mit je 500 ml $H_2O$ gewaschen, der Durchlauf und die Waschphasen vereinigt und lyophilisiert.

C. Reinigung über Biogel P-10

Das Lyophilisat wurde in $H_2O$ aufgenommen und an einer Biogel P-10-Säule (2,5 x 100 cm) in zwei Portionen mit $H_2O$ als Eluationsmittel chromatographiert. Es wurden Fraktionen von jeweils 4 ml genommen. Die Detektion erfolgte mit dem Anthrontest für Hexosen und Carbazol für Uronsäuren.
Fraktionen 51 - 77, 78 - 120 und 120 - 153 wurden vereinigt und lyophilisiert.

6

| Auswaage: | |
|---|---|
| Fraktion I | 492 mg |
| Fraktion II | 786 mg |
| Fraktion III | 4.102 mg |

Die drei Fraktionen wurde auf immunstimulierende Wirkung getestet. Fraktion I ergab die höchste Wirkung.

D. Reinigung über QUAE-Sephadex A-25

360 ml QUAE-Sephadex A-25 wurden mit 0,15 M Ammoniumformiat-Puffer, pH 4,0 äquilibriert. Fraktion I der Chromatographie an Biogel P-10 wurde in dem gleichen Puffer aufgenommen, auf die Säule gegeben und mit einem Säulenvolumen Aufgabepuffer gewaschen (Flow 50 ml/h). Anschließend wurde ein linearer Salzgradient von 0,15 → 2 M Ammoniumformiat, pH 4,0, angelegt. Die Hauptkomponente (Anthrontest, Uronsäuretest) eluierte bei einer Ammoniumformiatkonzentration von ca. 0,77 M. Die entsprechenden Fraktionen wurden zusammengegeben und lyophilisiert.

E. Entsalzung über Biogel P-2

Die lyophilisierte Hauptkomponente der Anionenaustauscherchromatographie wurde in 50 ml $H_2O$ aufgenommen und durch Gelchromatographie an Biogel P-2-Säule (5 x 100 cm) mit einem Flow von 100 ml/h mit $H_2O$ eluiert. Die einzelnen Fraktionen von je 8,5 ml wurden mit dem Anthrontest auf Hexosen und durch Leitfähigkeitsmessungen auf Salzgehalt untersucht. Die kohlenhydrathaltigen Fraktionen (Fraktionen 60 - 80) wurden zusammengegeben und lyophilisiert.
Ausbeute: 93 mg
Dieses Glykopeptid wurde auf immunstimulierende Wirkung getestet.
Die erfindungsgemäß hergestellten Zellwandkomponenten weisen eine breite abwehrsteigernde Wirkung auf.
Substanzen, die die körpereigene Abwehr (Immunsystem, Phagocytose) während einer Infektion stimulieren, sind sowohl für die Human- wie für die Veterinärmedizin von großem Interesse, da viele Infektionen trotz guter chemotherapeutischer Möglichkeiten ohne Unterstützung durch körpereigene Abwehrmechanismen persistieren. Dies kann zum erneuten Auftreten von Symptomen (Rezidiv) nach dem Überstehen der Ersterkrankung und damit zu chronisch rezidivierenden Krankheiten führen. Unter den durch Bakterien bedingten Erkrankungen sind es besonders Infektionen mit fakultativ intrazellulären Bakterien, die Probleme darstellen.
Ein Experimentalmodell für eine solche Erkrankung stellt die Infektion der Maus mit Salmonella typhimurium dar.
Nach Inokulation der Mäuse mit diesen humanpathogenen Bakterien kommt es in Abhängigkeit von der Infektionsdosis zu einem subakut bis chronischen Krankheitsverlauf, bei dem die Tiere erst nach 4 bis 7 Tagen abzusterben beginnen. Während dieses Zeitraumes besteht die Möglichkeit, das Immunsystem durch Substanzen zu beeinflussen. Während der ersten zwei Wochen werden hohe Keimzahlen im Blut und in Leber und Milz infizierter Tiere gefunden. Die Keimzahlen nehmen danach allmählich ab, sind aber noch 8-12 Wochen nach der Inokulation nachweisbar. Bei den meisten anderen tierexperimentellen Infektionen, kommt es zu einem sehr schnellen Absterben der Tiere innerhalb von 1 bis 2 Tagen. Damit besteht keine Möglichkeit mehr, die Abwehr während der Infektion zu stimulieren.
Es ist ferner bekannt, daß N-Acetyl-muramyl-L-alanyl-D-isoglutamin, die kleinste wirksam Komponente aus der Zellwand von Mykobakterien, die unspezifische Infektionsabwehr stimuliert (Robert Koch Stiftung e.V., Beiträge und Mitteilungen Vol. 5/1983, S. 31-38).
Es wurde nun überraschenderweise gefunden, daß auch die erfindungsgemäß hergestellten Zellwandkomponenten die unspezifische Abwehr gegenüber Infektionen steigern kann. Dies wurde anhand der folgenden Versuche gefunden:

1. Keimzahlreduktion

Die Zellwandkomponenten wurden Mäusen einmalig vor der Infektion entweder intraperitoneal oder subcutan in unterschiedlichen Dosen verabfolgt, und zwar 1 Tag vor subcutaner Infektion mit $2 \times 10^5$ Kolonien-bildenden Einheiten (KbE) von Salmonella typhimurium. Diese Infektionsdosis führt bei unbehan-

7

delten Tieren am 3. Tag zu einer hohen Keimzahl im Blut und in den Organen, insbesondere Leber und Milz. Die Tiere wurden in Makrolon-Käfigen unter konstanten Bedingungen (22° ± 2°C; 55-65 % relative Luftfeuchtigkeit) gehalten und erhielten Sniff Versuchstierdiät.

In mehreren Versuchen kam es nach Behandlung der Tiere mit Zellwandkomponenten in Dosierungen von 0,1, 1,10 oder 100 mg/kg Wirkstoff zu einer signifikanten Verminderung der Keimzahlen im Blut infizierter Mäuse im Vergleich zu Tieren, die nicht behandelt worden waren.

2. Letalität

Die Substanzen der Erfindung bewirken nach einmaliger subcutaner Gabe von 0,1, 1,0 oder 10 und 25 mg/kg vor letaler Infektion mit Salmonella typhimurium eine signifikante Erhöhung der Überlebensrate, die noch am 42. Tag post infectionem nachweisbar ist.

Bei Mäusen, die mit der 10-fachen $LD_{50}$ der Bakterien infiziert worden waren, kam es durch die Behandlung zu einer signifikanten Erhöhung der Überlebensrate und einer Verlängerung der Überlebenszeit im Vergleich zu unbehandelten Tieren (s. Tabelle).

## Tabelle

### Wirkung von Komponenten aus Archaebakterien (Halobacterium salinarium) im Infektionsmodell

|  | ZWK 56/2 I | | ZWK 71 | |
|---|---|---|---|---|
| Dosis[a] mg/kg | Mittlere Überle- benszeit (Tage) | Überle- bens- rate (Tag 42) | Mittlere Überle- benszeit (Tage) | Überle- bens- rate (Tag 42) |
| 0,1 | 38 | 12/24[b] | 23 | 7/24 |
| 1,0 | >42 | 15/24[c] | 28 | 11/24[b] |
| 10,0 | >42 | 17/24[c] | >42 | 14/24[c] |
| Kontrolle | 7,5 | 4/24 | 7,5 | 4/24 |

a) Einmalige subcutane Gabe 24 Stunden vor letaler Infektion mit Salmonella typhimurium

b) p <0,05, Fisher Test.

c) p <0,005, Fisher Test.

Diese Wirkungen wurden bei parenteraler Gabe der Substanzen gefunden. Sie führen bei parenteraler Gabe zu einer deutlichen Verminderung der Bakterienzahlen im Blut und in der Leber und zwar nach intraperitonealer Infektion mit sog. intrazellulären Bakterien, d.h. Bakterien, die sich nach Aufnahme in die Makrophagen - den wichtigsten Zellen der unspezifischen Abwehr - so lange weitervermehren, bis diese Zellen im Immunsystem aktiviert und damit in die Lage versetzt werden, die Bakterien intrazellulär abzutöten.

3. Granulozytenaktivität

Die Phagozyten sind ein wichtiger Bestandteil der unspezifischen Infektionsabwehr. Sie sind in der Lage, sehr früh in das Infektionsgeschehen einzugreifen und invasierende Erreger abzuwehren. Eine wichtige Rolle spielen hierbei die über den oxidativen Metabolismus der Phagozyten entstandenen toxischen und reaktiven Sauerstoffspezies ($O_2^-$, $H_2O_2$, OH, $^1O_2$), die antimikrobielle Eigenschaften besitzen.

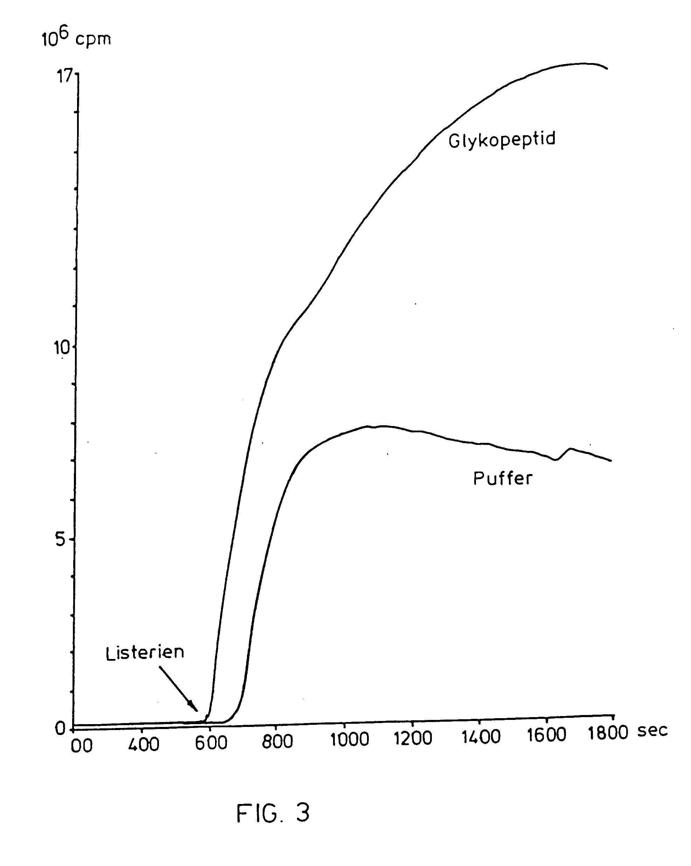

Mit der Methode der Luminol-abhängigen Chemilumineszenz-Messung ist es möglich, Aktivitätsänderungen des oxidativen Metabolismus der Phagozyten unter verschiedenen Bedingungen, so auch unter Substanzeinfluß zu überprüfen. Entsprechende Studien wurden an humanen, neutrophilen Granulozyten aus peripherem Blut vorgenommen: Es wurde der direkte Einfluß des Glykoproteins aus Archaebakterien auf Humangranulozyten und die Wirkung der Substanz auf die durch Listerien induzierte Granulozytenaktivität untersucht. Das Glykoprotein alleine zeigte keine Wirkung auf die Granulozytenaktivität. Die durch Listerien induzierte Chemilumineszenz von Granulozyten wurde jedoch in Gegenwart des Glykoproteins 100 $\mu$g/$10^6$ Zellen/ml deutlich verstärkt (Abb. 1). Schon bei einer Konzentration von 0,01 $\mu$g/$10^6$ Zellen/ml konnten wenn auch geringe Wirkung auf die Granulozytenaktivität beobachtet werden (Abb. 3).

Die pharmazeutischen Präparate der vorliegenden Erfindung sind vorzugsweise Tabletten oder Gelatinekapseln, welche die Wirkstoffe zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salze davon, wie Magnesium- oder Calciumstearat, und/oder Polyethylenglykol, enthalten. Tabletten enthalten ebenfalls Bindemittel, z.B. Magnesium-aluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmacksstoffe und Süßmittel.

Injizierbare Präparate sind vorzugsweise isotonische wäßrige Lösungen oder Suspensionen. Suppositorien, Salben oder Cremes sind in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1 % bis etwa 75 % insbesondere von etwa 1 % bis 50 % der genannten Aktivstoffe.

**Patentansprüche**

**Patentansprüche für folgende Vertragsstaaten : AT, BE, CH, DE, FR, GB, IT, LI, NL, SE**

1. Arzneimittel, enthaltend Zellwandkomponenten aus Kulturen bzw. Zellwänden von Halobakterien salinarium oder Halobakterium halobium, gewonnen aus Kulturen der Halobakterien, wobei man die Halobakterien anzüchtet, das Zellwandprotein der Halobakterien isoliert, das Zellwandprotein enzymatisch abbaut, und die Abbauprodukte reinigt und isoliert, wobei die Auswahl der Fraktionen bei den einzelnen Reinigungsschritten ausschließlich aufgrund der immunstimulierenden Aktivität der Fraktionen erfolgt.

2. Arzneimittel nach Anspruch 1, dadurch gekennzeichnet, daß man das Zellwandprotein tryptisch abbaut und die dabei erhaltene Glycopeptidfraktion mit Subtilisin abbaut.

**Patentansprüche für folgenden Vertragsstaat : ES**

1. Verfahren zur Herstellung von Arzneimitteln, enthaltend Zellwandkomponenten aus Kulturen bzw. Zellwänden von Halobakterien salinarium oder Halobakterium halobium, gewonnen aus Kulturen der Halobakterien, wobei man die Halobakterien anzüchtet, das Zellwandprotein der Halobakterien isoliert, das Zellwandprotein enzymatisch abbaut, und die Abbauprodukte reinigt und isoliert, wobei die Auswahl der Fraktionen bei den einzelnen Reinigungsschritten ausschließlich aufgrund der immunstimulierenden Aktivität der Fraktionen erfolgt.

**2.** Verfahren zur Herstellung von Arzneimitteln nach Anspruch 1, dadurch gekennzeichnet, daß man das Zellwandprotein tryptisch abbaut und die dabei erhaltene Glycopeptidfraktion mit Subtilisin abbaut.

## Claims

**Claims for the following Contracting States : AT, BE, CH, DE, FR, GB, IT, LI, NL, SE**

**1.** Medicaments containing cell wall components from cultures or cell walls of Halobacterium salinarium or Halobacterium halobium, obtained from cultures of Halobacteria, the Halobacteria being cultured, the cell wall protein of the Halobacteria being isolated, the cell wall protein being enzymatically degraded, and the degradation products being purified and isolated, with the selection of the fractions in the individual purification steps being based exclusively on the immunostimulating activity of the fractions.

**2.** Medicaments according to Claim 1, characterised in that the cell wall protein is degraded by trypsin, and the glycopeptide fraction obtained thereby is degraded with subtilisin.

**Claims for the following Contracting State : ES**

**1.** Process for the preparation of medicaments containing cell wall components from cultures or cell walls of Halobacterium salinarium or Halobacterium halobium, obtained from cultures of Halobacteria, in which the Halobacteria are cultured, the cell wall protein of the Halobacteria is isolated, the cell wall protein is enzymatically degraded, and the degradation products are purified and isolated, with the selection of the fractions in the individual purification steps being based exclusively on the immunostimulating activity of the fractions.

**2.** Process for the preparation of medicaments according to Claim 1, characterised in that the cell wall protein is degraded by trypsin, and the glycopeptide fraction obtained thereby is degraded with subtilisin.

## Revendications

**Revendications pour les Etats contractants suivants : AT, BE, CH, DE, FR, GB, IT, LI, NL, SE**

**1.** Médicaments contenant des composants de parois cellulaires provenant de cultures ou de parois cellulaires de Halobacterium salinarium ou Halobacterium halobium, extraits de cultures des halobactéries, les halobactéries étant cultivées, la protéine de la paroi cellulaire des halobactéries isolée, la protéine de paroi cellulaire dégradée enzymatiquement et les produits de dégradation purifiés et isolés, les fractions étant sélectionnées au cours des différentes étapes de purification exclusivement d'après leur activité immunostimulante.

**2.** Médicaments selon la revendication 1, caractérisés en ce qu'on dégrade trypsiquement la protéine de paroi cellulaire et qu'on dégrade la fraction glycopeptidique ainsi obtenue à l'aide de subtilisine.

**Revendications pour l'Etat contractant suivant : ES**

**1.** Procédé de préparation de médicaments contenant des composants de parois cellulaires provenant de cultures ou de parois cellulaires de Halobacterium salinarium ou Halobacterium halobium, extraits de cultures des halobactéries, les halobactéries étant cultivées, la protéine de la paroi cellulaire des halobactéries est isolée, la protéine de paroi cellulaire dégradée enzymatiquement et les produits de dégradation purifiés et isolés, les fractions étant sélectionnées au cours des différentes étapes de purification uniquement d'après leur activité immunostimulante.

**2.** Procédé de préparation de médicaments selon la revendication 1, caractérisé en ce qu'on dégrade tryptiquement la protéine de paroi cellulaire et qu'on dégrade la fraction glycopeptidique ainsi obtenue à l'aide de subtilisine.

FIG. 1

EP 0 286 869 B1

FIG. 2

FIG. 3